(19) Europäisches Patentamt
European Patent Office
Office européen des brevets

(11) Publication number : **0 501 641 A1**

(12) **EUROPEAN PATENT APPLICATION**

(21) Application number : **92301270.2**

(51) Int. Cl.$^5$ : **A61K 31/425,** A61K 37/02

(22) Date of filing : **17.02.92**

(30) Priority : **26.02.91 US 660791**

(43) Date of publication of application :
**02.09.92 Bulletin 92/36**

(84) Designated Contracting States :
**BE CH DE ES FR GB IT LI SE**

(71) Applicant : **CLINTEC NUTRITION COMPANY**
**Three Parkway North, Suite 500**
**Deerfield, Illinois 60015 (US)**

(72) Inventor : **Trimbo, Susan**
**737 Ridge Avenue**
**Evanston, Illinois 60202 (US)**
Inventor : **Goldberg, Dennis I.**
**920 Countryside Drive**
**Palatine, Illinois 60067 (US)**

(74) Representative : **MacGregor, Gordon**
**ERIC POTTER & CLARKSON St. Mary's Court**
**St. Mary's Gate**
**Nottingham, NG1 1LE (GB)**

(54) **L-2-oxothiazolidine-4-carboxylate and glutathion esters for the treatment of hepatic diseases.**

(57) The use of a composition that stimulates the intracellular synthesis of glutathione for providing a composition for treating hepatic disease is provided. In an embodiment, composition, that is administered to a patient having hepatic disease, includes a compound chosen from the group consisting of : L-2-oxothiazolidine-4-carboxylate ; glutathione esters ; and thiazolidine-4-carboxylate analogs, in an amount sufficient to stimulate intracellular glutathione synthesis. The compound can be administered parenterally or enterally.

EP 0 501 641 A1

## BACKGROUND OF THE INVENTION

The present invention relates generally to the treatment of hepatic disease and associated lung and kidney diseases.

Liver or hepatic disease has numerous potential causes. For example, hepatic failure can be caused by traumatic injury to the organ as well as metabolic causes of a chronic nature, e.g. alcoholism, or acute nature, e.g., hepatitis or sepsis. The liver has many essential functions. For example, the liver is the site of detoxification of numerous substances. Nitrogenous wastes associated with piotein metabolism are detoxified in the liver. An especially important toxic by-product is ammonia. The liver normally detoxifies ammonia by forming a nitrogen-containing substance, urea, which is then excreted via the kidneys.

The liver is also the primary site for detoxification of electrophilic metabolites and reactive oxygen intermediates generated during the metabolism of xenobiotics. Much of this detoxification is achieved by conjugation with glutathione and subsequent excretion inbile. In addition, the liver is the main source of glutathione synthesis and secretion, supplying glutathione for high use organs such as the lungs and kidneys.

Hepatic failure, due to a variety of disease states, can compromise synthesis of glutathione in the liver. This makes the liver, and the peripheral tissues, more susceptible to oxidative damage. In cirrhosis, cell damage due to oxidative stress can be widespread in the liver.

Cirrhosis is defined as the disorganization of the liver architecture by widespread fibrosis resulting in nodule formation. See Merck Manual, 15th Edition. Cirrhosis is the end product of the reaction of the liver to certain injuries with the resulting morphology not related so much to the injurious agent as to the form of injury and the liver's reaction to it. Fibrosis and parenchymal regeneration result as the natural, but modifiable reaction to this injury. Merck Manual, 15th Edition.

Cirrhotic patients exhibit many characteristics that result in future damage to the liver and/or risk to the patient. For example, Olomu, et al., High Incidence of Poor Sulfoxidation in Patients with Primary Biliary Cirrhosis, the New England Journal of Medicine, Vol. 318, No. 17, (1988) pp. 1089-1092, found that in patients with biliary cirrhosis poor sulfoxidation was observed. Specifically, the authors studied the ability to sulfoxidate the amino acid analogue S-carboxmethyl-cysteine.

It has also been noted that cirrhotic patients have depressed levels of certain amino acids. Chawla, et al., Plasma Concentrations of Transsulfuration Pathway Products During Nasoenteral and Intravenous Hyperalimentation of Malnourished Patients, the American Journal of Clinical Nutrition, 42: October 1985, p. 577-584, found in cirrhotic cases eating a mixed diet, levels of taurine, cysteine, plasma glutathione and free choline were subnormal. During nasoenteral hyperalimentation, methionine was elevated while cysteine, glutathione and free choline remained depressed. Levels of taurine, cysteine, protein-bound cysteine, glutathione, free choline, and phosphatidyl choline remained depressed during TPN and methionine was again elevated.

Rudman, et al., Hypotyrosinemia, Hypocystinemia, and Failure to Retain Nitrogen During Total Parenteral Nutrition of Cirrhotic Patients, Gastroenterology, 1981: 81: 1025-345, concludes that in the cirrhotic patients studied nutritional repletion was blocked by deficiencies of cysteine and tyrosine, resulting from hepatic inability to synthesize cystine from methionine and tyrosine from phenylalanine.

Davis, et al., Relationship Between Hepatic Glutathione and Fatty Liver In Weanling Rats on a Low Protein Diet, Nutrition Reports International, April 1988, Vol . 37, No. 4, pp 847-856, notes that a low dietary cysteine can cause fatty infiltration of the liver and this may be mediated through a fall in liver glutathione.

Bauman, et al., Effect of Dietary Protein Deficiency and L-2-oxothiazolidine-4-carboxylate on the Diurnal Rhythm of Hepaic Glutathione in the Rat, American Institute of Nutrition 1988, pp 1048-1054, concludes that in addition to cysteine availability, previous dietary protein status plays a key role in the regulation of the feeding-induced diurnal rhythm of hepatic glutathione concentration in rats.

## SUMMARY OF THE INVENTION

The present invention relates to the use of a composition that stimulates the intracellular synthesis of glutathione for manufacturing a composition.

It has been found that by increasing glutathione levels within the liver cells, the liver cells will experience reduced oxidative stress during episodes of hepatic failure caused by, for example, liver cirrhosis. Reducing damage due to oxidative stress assists in overcoming the hepatic failure event, as well as reduces long term damage to the hepatic tissue. Increasing glutathione in the lungs and kidneys helps protect the organs against oxidative stress and membrane damage associated with liver disease.

In an embodiment of the present invention, L-2-oxothiazolidine-4-carboxylate is used to elevate tissue glutathione levels.

In another embodiment, glutathione esters are utilized to elevate glutathione levels within the cells. In a

preferred embodiment, the methyl ester or ethyl ester of glutathione is used. In an embodiment, glutathione isopropyl ester is used.

In an embodiment of the method the composition includes short chain acetyl esters of glutathione.

Additional features and advantages of the present invention are described in, and will be apparent from, the Detailed Description of the Presently Preferred Embodiments.

## DETAILED DESCRIPTION OF THE PRESENTLY PREFERRED EMBODIMENTS

The present invention provides the use of a composition that stimulates the intracellular synthesis of glutathione for creating a composition for treating hepatic disease. Specifically, the present invention provides a method for treating hepatic failure by reducing damage to hepatic, lung, and kidney cells due to oxidative stress.

Pursuant to the present invention, the composition increases the intracellular glutathione levels of the hepatic cells of a patient with hepatic disease. It has been found that in hepatic disease patients, the glutathione levels are suppressed. Indeed, one of the principal sites of production of glutathione is in the liver of the patient. Accordingly, hepatic failure and disease results in substantially decreased intracellular levels of glutathione. Due to the suppressed levels of glutathione, oxidative stress, that can occur due to cirrhosis or other liver diseases, can damage the hepatic cells.

Decreased secretion of glutathione also compromises glutathione levels in high use organs such as the lungs and kidneys, making these organs more susceptible to oxidative damage.

Pursuant to the present invention, a composition that is a precursor to the intracellular synthesis of glutathione is administered to a patient. The composition can be administered either enterally or parenterally.

Pursuant to the present invention any substrate that stimulates intracellular glutathione synthesis can be utilized. Preferably, the method comprises the step of administering a therapeutically effective amount of an agent chosen from the group consisting of L-2-oxothiazolidine-4-carboxylate and glutathione esters. However, other thiazolidine-4-carboxylate analogs that are converted intracellularly to glutathione can be utilized.

L-2-oxothiazolidine-4-carboxylate, in vivo, is subjected to the action of 5-oxo-L-prolinase in the presence of adenosine triphosphate to produce S-carboxyl cysteine. S-carboxyl cysteine is then decarboxylated to produce cysteine. Cysteine is then metabolized to provide glutathione. See, U.S. Patent Nos.: 4,335,210; 4,434,158; 4,438,124; 4,665,082; and 4,647,571, the disclosures of which are incorporated herein by reference.

As previously stated, the non-cysteine substrate can include a glutathione ester. For example, the compound can have the structure:

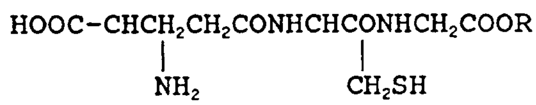

wherein: R is an alkyl group containing 1 to 10 carbon atoms. Preferably, the methyl and ethyl glutathione esters are used. Glutathione esters are disclosed in U.S. Patent No. 4,784,685, the disclosure of which is incorporated herein by reference.

In an embodiment of the invention, the composition is administered parenterally and includes 3% or 6%, by weight, L-2-oxothiazolidine-4-carboxylate in a buffer solution such as phosphate buffer.

In an embodiment, the composition is administered orally as a tablet or capsule and includes L-2-oxothiazolidine-4-carboxylate.

In an embodiment, the composition is administered orally in tablet or capsule form and includes glutathione ester.

In an embodiment, the composition is administered as part of a parenteral nutrition regimen and includes glutathione ester.

In an embodiment, the composition is administered as part of an enteral diet and includes glutathione ester.

The composition can be administered as an adjunct therapy with other typical therapies. For example, Travasorb® Hepatic marketed by Clintec Nutrition Company, Deerfield, Illinois, is an enteral diet used for the nutritional support of patients with liver failure. The diet is a defined formula diet high in branched-chain amino acids and low in aromatic and ammongenic amino acids with non-protein calories derived primarily from glucose oligosaccharides and from fat. The composition of the present invention can be administered enterally with the Travasorb® Hepatic diet or other enteral formulations.

By way of example, but not limitation, contemplated examples of the present invention will now be given.

**EXAMPLE 1 - Acute Exacerbation of Cirrhosis**

A 55 year old male presented with acute cirrhosis and Grade III encephalopathy. Total parenteral nutrition (TPN) was initiated to correct coexisting malnutrition. Amino acids were given at a dose of .75 g/kilogram body weight per day and were provided as a mixture of 4% BranchAmin® and 10% Travasol® such that the target branched chain amino acid content was 50%.

Procysteine™ (3% (w:v) L-2-oxothiazolidine-4-carboxylate in a phosphate buffer) was provided as part of the amino acid mixture at a level of .035 g/kilogram/day. Plasma amino acids and glutathione were monitored on Day 1 and Day 7.

Select findings are presented below:

| | Day 1 | Day 7 |
|---|---|---|
| Methionine (uM) | 140.1 | 35.6 |
| Cysteine (uM) | 5.7 | 10.5 |
| Glutathione (uM) | 1.0 | 3.5 |

**EXAMPLE 2 - Chronic Liver Disease - Nutritional Supplementation with Enteral diet containing Procysteine™**

A 52 year old female with chronic alcoholic cirrhosis and protein energy malnutrition was prescribed Travasorb® Hepatic containing Procysteine™ to improve nutritional and metabolic status. The daily diet provided 40 g amino acids/liter (50% branched chain amino acids, 2% aromatic amino acids and 1.0 g Procysteine, a cysteine precursor). The diet was given as a total enteral feeding (1.2 x REE).

Improvements in metabolic status were noted by week 2. Normalization of plasma amino acid profiles and plasma glutathione were observed. Values are given below:

| | Day 1 | Day 14 |
|---|---|---|
| Amino Acids (umol/ml) | | |
| Leu | .092 | .090 |
| Iso | .005 | .063 |
| Val | .235 | .210 |
| Met | .044 | .015 |
| Tyr | .063 | .040 |
| Phe | .152 | .054 |
| Plasma Glutathione ($\mu$M) | .95 | 4.2 |

**EXAMPLE 3 - Post Transplant Supplementation with Glutathione Esters**

A 59 year old male with primary biliary cirrhosis underwent liver transplant. In addition to routine post-surgery immuno-suppressive and nutritional therapies the patient received a glutathione-containing tablet t.i.d. containing 0.5 g glutathione methyl ester. The goal of the therapy was to normalize plasma and tissue glutathione status.

Plasma glutathione concentration was monitored periodically; values for Week 1 and Week 12 are given below:

| | Week 1 | Week 12 | Normal Range |
|---|---|---|---|
| Plasma | | | |
| Glutathione (uM) | 1.2 | 3.9 | 4.5 |

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. The use in the preparation of a composition for treating hepatic disease in a patient suffering from same of an agent that stimulates the intracellular synthesis of glutathione in an amount sufficient to increase intracellular glutathione levels of at least the hepatic cells.

2. The use according to Claim 1 wherein the composition is parenterally administrable.

3. The use according to Claim 1 wherein the composition is enterally administrable.

4. The use according to Claim 1, 2 or 3 wherein the agent includes L-2-oxothiazolidine-4-carboxylate.

5. The use according to Claim 1, 2 or 3 wherein the agent includes a glutathione ester.

6. The use according to Claim 1, 2 or 3 wherein the agent includes a thiazolidine-4-carboxylate analog.

7. The use according to Claim 1, 2 or 3 wherein the agent includes a methyl ester of glutathione.

8. The use according to Claim 1, 2 or 3 wherein the agent includes an ethyl ester of glutathione.

9. The use according to Claim 1, 2 or 3 wherein the agent includes short chain actyl esters of glutathione.

10. The use according to Claim 1, 2 or 3 wherein the agent includes a glutathione isopropyl ester.

European Patent Office

# EUROPEAN SEARCH REPORT

Application Number

EP 92 30 1270

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | US-A-4 665 082 (MEISTER et al.) * Column 1, lines 15-22; column 4, line 20 - column 5, line 35; claims * --- | 1,2,4,6 | A 61 K 31/425<br>A 61 K 37/02 |
| D,X | US-A-4 784 685 (MEISTER) * Column 3, line 10 - column 4, line 10; claims * --- | 1,2,5,7-10 | |
| X | US-A-4 868 114 (NAGASAWA et al.) * Column 1, lines 12-20; column 6, lines 31-39; claims * --- | 1,2,4,6 | |
| X | BIOCHEMICAL PHARMACOLOGY, vol. 39, no. 12, 15th June 1990, pages 1877-1881, Pergamon Press plc, GB; S. UHLIG et al.: "Glutathione enhancement3in various mouse organs and protection by glutathione isopropyl ester against liver injury" * Whole document * --- | 1,2,5,9,10 | |
| | | | TECHNICAL FIELDS SEARCHED (Int. Cl.5) |
| X | CANCER, vol. 62, no. 7, 1988, pages 1275-1281; B.A. TEICHER et al.: "Glutathione monoethyl ester can selectively protect liver from high dose BCNU or cyclophosphamide" * Whole document * --- | 1,2,5,8,9 | A 61 K |
| X | THE JOURNAL OF PHARMACOLOGY AND EXPERIMENTAL THERAPEUTICS, vol. 237, no. 1, 1986, pages 341-349, The American Society for Pharmacology and Experimental Therapeutcs, US; G.A. HAZELTON et al.: "Effects of cysteine pro-drugs on acetaminophen-induced hepatotoxicity" * Whole document * --- -/- | 1,2,4,6 | |

The present search report has been drawn up for all claims

| Place of search | Date of completion of the search | Examiner |
|---|---|---|
| THE HAGUE | 15-04-1992 | GOETZ G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Page 2

Application Number

EP 92 30 1270

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | PHYSIOLOGICAL CHEMISTRY AND PHYSICS AND MEDICAL N M R, vol. 19, 1987, pages 9-13; H. NISHINA et al.: "Effect of L-2-oxothiazolidine-4-carboxylate administration glutathione and cysteine concentrations in guinea pig liver and kidney" * Whole document * --- | 1,2,4,6 | |
| X | J. NUTRITION, vol. 120, no. 2, February 1990, pages 158-165, American Institute of Nutrition; T.K. CHUNG et al.: "L-2-oxothiazolidine-4-carboxylate as a cysteine precursor: Efficacy for growth and hepatic glutathione synthesis in chicks and rats" * Whole document * ----- | 1,2,3,4,6 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 15-04-1992 | GOETZ G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)